# EUROPÄISCHE PATENTSCHRIFT

(11) **EP 0 607 803 B1**
(45) Veröffentlichungstag und Bekanntmachung des Hinweises auf die Patenterteilung: **10.07.1996**
(21) Anmeldenummer: 94100106.7
(22) Anmeldetag: 05.01.1994
(51) Int. Cl.: C07D 213/85

(54) **Verfahren zur Herstellung von 6-Amino-nicotinsäurenitrilen**
Process for the preparation of 6-amino-3-pyridinecarbonitriles
Procédé pour la préparation de 6-amino-3-pyridinecarbonitriles

(30) Priorität: 18.01.1993 DE 4301109
(43) Veröffentlichungstag der Anmeldung: 27.07.1994
(73) Patentinhaber: BAYER AG, 51368 Leverkusen (DE)
(72) Erfinder: Kraus, Helmut, Dr., D-51519 Odenthal (DE)

(56) Entgegenhaltungen:
- EP-A- 0 060 071
- SCIENCE Bd. 166, Nr. 3901 , 3. Oktober 1969 , LANCASTER, PA US Seiten 765 - 766 J.P. FERRIS ET AL. 'Photochemical reactions and the chemical evolution of purines and nicotinamide derivatives'

## Beschreibung

Die Erfindung betrifft ein Verfahren zur Herstellung von 6-Amino-nicotinsäurenitrilen (2-Amino-5-cyano-pyridinen) über die intermediäre Zwischenstufe der 2-Imino-5-cyano-1,2-dihydropyridine durch Umsetzung von Methylen-glutaconsäuredinitrilen mit primären Aminen, Hydrazinen bzw. Ammoniak.

Die erfindungsgemäß herstellbaren Heterocyclen sind Zwischenprodukte zur Herstellung pharmazeutischer und agrochemischer Wirkstoffe (EP 181 636, JP 61/257 960 (1986)). Da sich 2-Amino-pyridine leicht zu den entsprechenden Halogenverbindungen umsetzen lassen (GB 1.215.387), stellt 6-Amino-nicotinsäurenitril ein wichtiges Zwischenprodukt zur Synthese von Insektiziden der Nitromethylen-Klasse dar (EP 235 725, EP 383 091, DE-OS 37 26 993).

2-Amino-pyridine lassen sich aus den entsprechend substituierten Ausgangsstoffen nach Tschitschibabin mit Natriumamiden in flüssigem Ammoniak herstellen. Allerdings ist diese Methode meist mit dem Problem der Entstehung von unerwünschten Isomeren behaftet. Die gezielte Synthese durch Ringschluß-Reaktion von geeigneten Nitrilen mit Aminen ist für einige wenige substituierte Pyridine in guten Ausbeuten möglich (The Chemistry of Heterocyclic Compounds (G.R. New Kome), Interscience/John Wiley, Teil 5, Pyridin, S. 118). Weiterhin kann 2-Amino-5-phenyl-pyridin, ausgehend von 4-Dimethylamino-3-phenyl-pentadien-(1,3)-nitril mit Ammoniak in Dimethylsulfoxid hergestellt werden (EP 60 071); jedoch erhält man hierbei aus 3 g Ausgangsmaterial nur 300 mg des gewünschten Produktes.

6-Amino-nicotinsäurenitril ist durch Belichten von 2-Formyl-glutaconsäuredinitril in 1 molarer ammoniakalischer Lösung in 10 %iger Ausbeute zugänglich (Science 166, 765), während eine rein thermische Synthese nur mit noch geringerer Ausbeute möglich ist (J. Mol. Biol. 33 (1968), 693, gemäß Zitat in Science, loc. cit.).

5-Cyano-1,2-dihydro-2-imino-1-methylpyridin kann, ausgehend von 2-Amino-5-cyanopyridin durch Methylierung mit Methyliodid und anschließender Freisetzung mit Alkali hergestellt werden (J. Chem. Soc. 1965, 5542).

Bei Betrachtung dieses Standes der Technik ist es überraschend, daß sich Methylen-glutaconsäuredinitrile in hohen Ausbeuten zu den entsprechenden 2,5-disubstituierten Pyridinkörpern umsetzen lassen.

Die Erfindung betrifft demnach ein Verfahren zur Herstellung von 6-Amino-nicotinsäurenitrilen der Formel 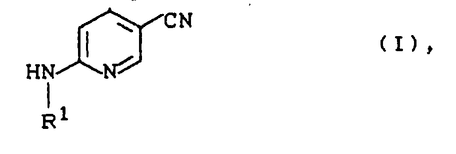 worin
- R¹: Wasserstoff, geradkettiges oder verzweigtes C₁-C₈-Alkyl, C₃-C₈-Alkenyl, C₂-C₈-Alkoxyalkyl, C₄-C₈-Alkoxyalkenyl, C₃-C₈-Cycloalkyl, C₇-C₁₀-Aralkyl oder -N(R²R³) bedeutet,
wobei R² und R³ unabhängig voneinander Wasserstoff, geradkettiges oder verzweigtes C₁-C₈-Alkyl, Phenyl, Tolyl, C₇-C₁₀-Aralkyl oder einen 5-bis 8-gliedrigen gesättigten oder ungesättigten heterocyclischen Ring, dessen Heteroatome 1 oder 2 aus der Gruppe von N, O und S sind, bedeuten, wobei weiterhin R² und R³ gemeinsam mit dem N-Atom, an das sie gebunden sind, einen 5- bis 8-gliedrigen Ring bilden können, der ein weiteres Heteroatom aus der Gruppe von N, O und S enthalten kann,
das dadurch gekennzeichnet ist, daß man Methylenglutaconsäuredinitrile der Formel 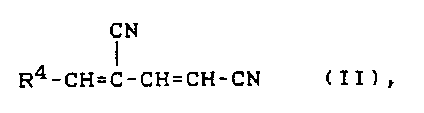 in der
- R⁴: für -OR⁵ oder -N(R⁵,R⁶) steht,
worin R⁵ und R⁶ geradkettiges oder verzweigtes C₁-C₈-Alkyl, C₃-C₈-Alkenyl, C₂-C₈-Alkoxyalkyl, C₄-C₈-Alkoxyalkenyl, C₃-C₈-Cycloalkyl oder C₇-C₁₀-Aralkyl bedeuten, wobei R⁵ und R⁶ weiterhin gemeinsam mit dem N-Atom, an das sie gebunden sind, einen 5-bis 8-gliedrigen Ring bilden können, der ein weiteres Heteroatom aus der Gruppe von N, O und S enthalten kann,
mit Stickstoffverbindungen der Formel

R¹-NH₂ (III),

worin
- R¹: die obige Bedeutung hat,
bei einer Temperatur von 0 bis 200°C, bevorzugt 20 bis 150°C und bei einem molaren Verhältnis von Stickstoffverbindung zu Glutaconsäuredinitril von 1 bis 100:1, bevorzugt 1 bis 10:1, und in Gegenwart oder in Abwesenheit eines inerten Lösungsmittels umsetzt.

Gemäß Bedeutungsumfang von R¹ wird die Umsetzung des Glutacondinitrils also mit Ammoniak, einem primären Amin oder einem Hydrazin durchgeführt.

Geradkettiges oder verzweigtes C₁-C₈-Alkyl ist beispielsweise Methyl, Ethyl, Propyl, Isopropyl, Butyl, Isobutyl, tert.-Butyl, die isomeren Amyle, Hexyle, Octyle, bevorzugt die genannten C₁-C₄-Alkylreste.

C₃-C₈-Alkenyl ist Allyl, die isomeren Butenyle, Amylenyle, Hexenyle oder Octenyle, bevorzugt die genannten C₃-C₄-Alkenylreste.

C₂-C₈-Alkoxyalkyl ist beispielsweise Methoxymethyl, Ethoxymethyl, Methoxyethyl sowie weitere Reste aus der Gruppe C₃-C₉-Alkyl, in welchem eine CH₂-Gruppe durch ein O-Atom ersetzt ist.

C₄-C₈-Alkoxyalkenyl ist beispielsweise Methoxyallyl, 2-Methoxy-propenyl und andere aus der Gruppe von C₄-C₉-Alkenyl, worin eine CH₂-Gruppe durch ein O-Atom ersetzt ist.

C₃-C₈-Cycloalkyl ist beispielsweise Cyclopropyl, Methyl-cyclopropyl, Dimethyl-cyclopropyl, Cyclobutyl, Methyl-cyclobutyl, Cyclopentyl, Methyl-cyclopentyl, Cyclohexyl, Methyl-cyclohexyl, Dimethyl-cyclohexyl, Cycloheptyl, Cyclooctyl, bevorzugt Cyclopropyl, Cyclopentyl und Cyclohexyl, sowie deren Methyl- oder Dimethyl-Derivate.

C₇-C₁₀-Aralkyl ist beispielsweise Benzyl, 1-Phenylethyl, 2-Phenylethyl und weitere dem Fachmann bekannte Reste dieser Art, bevorzugt Benzyl.

Als 5- bis 8-gliedriger gesättigter oder ungesättigter heterocyclischer Ring, dessen Heteroatome 1 oder 2 aus der Gruppe von N, O und S sind, seien genannt: Pyrrol, Furan, Thiophen, Pyrrolidin, Pyrazol, Imidazol, Thiazol, Oxazol, Pyridin, Pyrimidin, Piperazin, Morpholin, Pyran, Azepin, Azocin, Isoxazol, Isothiazol, Pyridazin und Pyrazin. Es ist dem Fachmann bekannt, daß ungesättigte heterocyclische Ringe einen mehr oder weniger stark ausgeprägten aromatischen Charakter haben können.

Weiterhin können R² und R³ gemeinsam mit dem N-Atom, an das sie gebunden sind, einen 5- bis 8-gliedrigen Ring bilden, der ein weiteres Heteroatom aus der Gruppe N, O und S enthalten kann. Solche Systeme sind beispielsweise, Pyrrolidin, Pyrrolin, Pyrazolidin, Imidazolidin, Thiazolidin, Piperazin, Piperidin, Morpholin, Azepin, Dihydroazocin.

In bevorzugter Weise werden im erfindungsgemäßen Verfahren Methylen-glutaconsäuredinitrile eingesetzt, in denen an die Stelle von R², R³ die Substituenten R¹², R¹³ treten, die unabhängig voneinander Wasserstoff, geradkettiges oder verzweigtes C₁-C₈-Alkyl, Cyclopropyl, Cyclopentyl, Cyclohexyl, Phenyl oder Benzyl bedeuten, wobei weiterhin R¹² und R¹³ gemeinsam mit dem N-Atom, an das sie gebunden sind, einen 5-bis 8-gliedrigen Ring bilden können, der ein weiteres Heteroatom aus der Gruppe von N, O und S enthalten kann.

In besonders bevorzugter Weise werden im erfindungsgemäßen Verfahren Glutaconsäuredinitrile eingesetzt, in denen an die Stelle von R¹², R¹³ die Substituenten R²², R²³ treten, die unabhängig voneinander Waserstoff, C₁-C₄-Alkyl oder Benzyl bedeuten, wobei weiterhin R²² und R²³ gemeinsam mit dem N-Atom, an das sie gebunden sind, Morpholin, Pyrrolidin oder Piperidin, das durch C₁-C₄-Alkyl oder durch Hydroxy-C₁-C₄-alkyl substituiert sein kann, bedeuten.

In weiterhin bevorzugter Weise treten an die Stelle von R⁵ und R⁶ die Substituenten R¹⁵ und R¹⁶, die unabhängig voneinander geradkettiges oder verzweigtes C₁-C₈-Alkyl, Cyclopropyl, Cyclopentyl, Cyclohexyl, Benzyl oder einem 5- bis 8-gliedrigen gesättigten oder ungesättigten Ring bedeuten, der ein weiteres Heteroatom aus der Gruppe N, O und S enthalten kann.

In weiterhin besonders bevorzugter Weise treten an die Stelle von R¹⁵ und R¹⁶ die Substituenten R²⁵ und R²⁶, die unabhängig voneinander geradkettiges oder verzweigtes C₁-C₄-Alkyl oder Benzyl bedeuten.

Die Umsetzung mit Ammoniak führt zum 2-Amino-5-cyanopyridin, während die Umsetzung mit primären Aminen oder mit Hydrazinen zunächst zum 2-Imino-5-cyano-1,2-dihydropyridin führt. Das 1-Methylderivat solcher Dihydropyridine ist bekannt (J. Chem. Soc. loc. cit.) und ist eine bei Raumtemperatur stabile Substanz. Unter den erfindungsgemäßen Reaktionsbedingungen lagern sich diese Dihydropyridine jedoch zu den aromatischen N-substituierten 2-Amino-5-cyanopyridinen um. Eine solche Umlagerung ist als Dimroth-Umlagerung bekannt. Die Reaktion des erfindungsgemäßen Verfahrens kann beispielsweise wie folgt dargestellt werden:

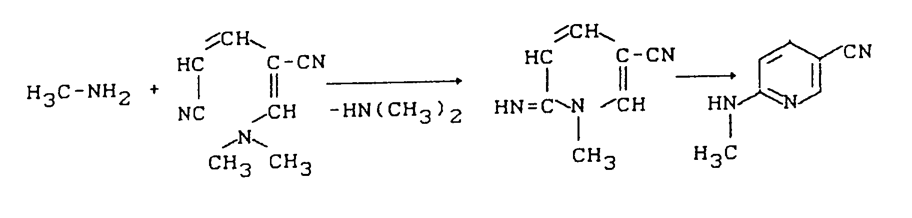

Die erfindungsgemäße Umsetzung kann in Lösung oder in Suspension durchgeführt werden. Als Reaktionsmedium kommen Kohlenwasserstoffe, halogenierte Kohlenwasserstoffe, Alkohole, Wasser, tert. Amine, Ketone, Nitrile, Dialkylcarboxamide, N-Alkyl-lactame, Peralkyl-harnstoffe, Dialkylsulfoxide, Dialkylsulfone, Ether, Phosphorsäureperalkylamide oder Gemische mehrerer aus den genannten Gruppen in Frage. Erfindungsgemäß kann ebenso in reinem oder verdünntem überschüssigen Ammoniak gearbeitet werden. Beispiele für Reaktionsmedien aus den genannten Gruppen sind: Petrolether, Toluol, Xylol, Cyclohexan, Chlortoluol, Ligroin, 1,2-Dichlorethan, Triethylamin, Aceton, Acetonitril, Dimethylacetamid, Dimethylformamid, N-Methyl-pyrrolidon, N-Methyl-caprolactam, Tetramethylharnstoff, Dimethylsulfoxid, Diethylsulfon, Methyl-tert.-butylether, Anisol, Tetrahydrofuran, Methanol, Isopropanol und Wasser.

Die Reaktionstemperatur liegt bei 0 bis 200°C, bevorzugt bei 20 bis 150°C. Die Zeitdauer der erfindungsgemäßen Umsetzung ist abhängig von der Ansatzgröße und von der Nucleophilie des Amins sowie von der Temperatur und kann zwischen 0,5 und 15 Stunden liegen.

Das molare Verhältnis von Stickstoffverbindung zu Glutaconsäuredinitril beträgt 1 bis 100:1, bevorzugt 1 bis 10:1. Die Stickstoffverbindung (III) kann teilweise oder vollständig auch als Salz einer Säure zum Einsatz kommen, beispielsweise in Form des Acetats.

Die einzusetzenden Methylen-glutaconsäuredinitrile sind beispielsweise durch Umsetzung von Glutaconsäuredinitril mit o-Estern erhältlich. Aminomethylen-glutaconsäuredinitrile können technisch einfach durch Dimerisierung von entsprechend substituierten β-Aminoacrylnitrilen erhalten werden.

### Beispiele

### Beispiel 1

73,5 g N,N'-Dimethylaminomethylen-glutaconsäuredinitril, 750 ml DMF und 136 g 25 %ige wäßrige Ammoniaklösung wurden in einem 1,3 l-Autoklaven 2,5 h auf 100°C erhitzt.

Das Reaktionsgemisch engte man am Rotationsverdampfer ein und erhielt 60 g Produkt als braunen Feststoff. Durch GC-Analyse (interner Standard) konnte ein Gehalt von 91,7 % an 2-Amino-5-cyanopyridin festgestellt werden, entsprechend 92,5 % der theoretischen Ausbeute. Nach Umkristallisation aus Wasser oder Isopropanol wurden Kristalle vom Schmelzpunkt 165°C erhalten.
¹H-NMR (d-Aceton) 6,2-6,6 ppm (s, 2H, NH₂), 6,65 ppm (d, 1H, H³), 7,67 ppm (d, 1H, H⁴), 8,30 ppm (s, 1H, H⁶).

### Beispiel 2

15 g des Dinitrils von Beispiel 1 und 7,7 g Ammoniumacetat wurden im Autoklaven vorgelegt. Bei Raumtemperatur leitete man 10,6 g Ammoniakgas ein und heizte 2 h auf 100°C. Nach Einengen und wäßriger Aufarbeitung wurde 2-Amino-5-cyanopyridin in 81,2 % der theoretischen Ausbeute erhalten.

### Beispiel 3

Auf 15 g des Dinitrils von Beispiel 1 wurden 70 g Ammoniakgas aufgedrückt. Nach 2 h bei 100°C entspannte man und erhielt 12,3 g 93,6 %iges Produkt, entsprechend 94,8 % der theoretischen Ausbeute.

### Beispiel 4

15 g Ethoxymethylen-glutaconsäuredinitril wurden analog Beispiel 3 mit Ammoniak cyclisiert. 2-Amino-5-cyanopyridin wurde in 85 % der Theorie erhaltene.

### Beispiel 5

7,4 g Dimethylaminomethylen-glutaconsäuredinitril, 100 ml DMF und 4,4 g 35,6 %ige wäßrige Methylaminlösung wurden in einen 0,3 1 V₄A-Autoklaven 2 Stunden bei 80°C gehalten. Nach Ausnehmen und Einengen wurden 6,4 g (93 %ige) 2-Methylamino-5-cyanopyridin erhalten, entsprechend 90,2 % der Theorie.

## Patentansprüche

1. Verfahren zur Herstellung von 6-Amino-nicotinsäurenitrilen der Formel 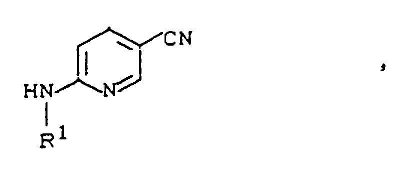 worin
R¹ Wasserstoff, geradkettiges oder verzweigtes C₁-C₈-Alkyl, C₃-C₈-Alkenyl, C₂-C₈-Alkoxyalkyl, C₄-C₈-Alkoxyalkenyl, C₃-C₈-Cycloalkyl, C₇-C₁₀-Aralkyl oder -N(R²R³) bedeutet,
wobei R² und R³ unabhängig voneinander Wasserstoff, geradkettiges oder verzweigtes C₁-C₈-Alkyl, Phenyl, Tolyl, C₇-C₁₀-Aralkyl oder einen 5- bis 8-gliedrigen gesättigten oder ungesättigten heterocyclischen Ring, dessen Heteroatome 1 oder 2 aus der Gruppe von N, O und S sind, bedeuten, wobei weiterhin R² und R³ gemeinsam mit dem N-Atom, an das sie gebunden sind, einen 5- bis 8-gliedrigen Ring bilden können, der ein weiteres Heteroatom aus der Gruppe von N, O und S enthalten kann,
dadurch gekennzeichnet, daß man Methylen-glutaconsäuredinitrile der Formel 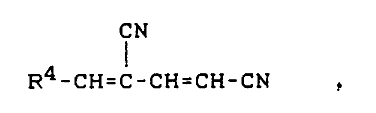 in der
R⁴ für -OR⁵ oder -N(R⁵,R⁶) steht,
worin R⁵ und R⁶ ein geradkettiges oder verzweigtes C₁-C₈-Alkyl, C₃-C₈-Alkenyl, C₂-C₈-Alkoxyalkenyl, C₄-C₈-Alkoxyalkenyl, C₃-C₈-Cyclaalkyl oder C₇-C₁₀-Aralkyl bedeuten, wobei R⁵ und R⁶ weiterhin gemeinsam mit dem N-Atom, an das sie gebunden sind, einen 5- bis 8-gliedrigen Ring bilden können, der ein weiteres Heteroatom aus der Gruppe von N, O und S enthalten kann,
mit Stickstoffverbindungen der Formel
R¹-NH₂,
worin
R¹ die obige Bedeutung hat,
bei einer Temperatur von 0 bis 200°C, bevorzugt 20 bis 150°C und bei einem molaren Verhältnis von Stickstoffverbindung zu Glutaconsäuredinitril von 1 bis 100:1, bevorzugt 1 bis 10:1 und in Gegenwart oder in Abwesenheit eines inerten Lösungsmittels umsetzt.

2. Verfahren nach Anspruch 1, dadurch gekennzeichnet, daß an die Stelle von R⁴ der Substituent R¹⁴ mit der Bedeutung -N(R⁵,R⁶) tritt.

3. Verfahren nach Anspruch 1, dadurch gekennzeichnet, daß an die Stelle von R¹ der Substituent R¹¹ mit der Bedeutung Wasserstoff, geradkettiges oder verzweigtes C₁-C₈-Alkyl, Cyclopropyl, Cyclopentyl, Cyclohexyl oder Benzyl tritt.

4. Verfahren nach Anspruch 3, dadurch gekennzeichnet, daß an die Stelle von R¹¹ der Substituent R²¹ mit der Bedeutung Wasserstoff, geradkettiges oder verzweigtes C₁-C₄-Alkyl oder Benzyl tritt.

5. Verfahren nach Anspruch 1, dadurch gekennzeichnet, daß an die Stelle von R² und R³ die Substituenten R¹² und R¹³ treten, die unabhängig voneinander Wasserstoff, geradkettiges oder verzweigtes C₁-C₈-Alkyl, Cyclopropyl, Cyclopentyl, Cyclohexyl, Phenyl oder Benzyl bedeuten, wobei beide Substituenten R¹² und R¹³ gemeinsam mit dem N-Atom, an das sie gebunden sind, einen 5- bis 8-gliedrigen Ring bilden können, der ein weiteres Heteroatom aus der Gruppe von N, O und S enthalten kann.

6. Verfahren nach Anspruch 5, dadurch gekennzeichnet, daß an die Stelle von R¹² und R¹³ die Substituenten R²² und R²³ treten, die unabhängig voneinander die Bedeutung Wasserstoff, C₁-C₄-Alkyl, Phenyl, Benzyl haben und wobei weiterhin die Substituenten R²² und R²³ gemeinsam mit dem N-Atom, an das sie gebunden sind, Morpholin, Pyrrolidin oder Piperidin, das durch C₁-C₄-Alkyl oder durch Hydroxy-C₁-C₄-alkyl substituiert sein kann, bilden können.

7. Verfahren nach Anspruch 1, dadurch gekennzeichnet, daß an die Stelle von R⁵ und R⁶ die Substituenten R¹⁵ und R¹⁶ treten, die unabhängig voneinander die Bedeutung geradkettiges oder verzweigtes C₁-C₈-Alkyl, Cyclopropyl, Cyclopentyl, Cyclohexyl, Phenyl, Benzyl oder eines 5- bis 8-gliedrigen gesättigten oder ungesättigten heterocyclischen Ringes haben, der ein weiteres Heteroatom aus der Gruppe von N, O und S enthalten kann.

8. Verfahren nach Anspruch 7, dadurch gekennzeichnet, daß an die Stelle von R¹⁵ und R¹⁶ die Substituenten R²⁵ und R²⁶ treten, die unabhängig voneinander die Bedeutung geradkettiges oder verzweigtes C₁-C₄-Alkyl, Phenyl oder Benzyl haben.

9. Verfahren nach Anspruch 1, dadurch gekennzeichnet, daß die Stickstoffverbindung teilweise oder vollständig in Form des Salzes einer Säure eingesetzt wird.

10. Verfahren nach Anspruch 1, dadurch gekennzeichnet, daß als Lösungsmittel eines oder mehrere aus der Gruppe der Kohlenwasserstoffe, halogenierten Kohlenwasserstoffe, tertiären Amine, Ketone, Nitrile, Dialkylcarboxamide, N-Alkyl-lactame, Peralkylharnstoffe, Dialkylsulfoxide, Dialkylsulfone, Sulfolane, Ether, Phosphorsäureperalkylamide, Alkohole, Wasser, bevorzugt eines oder mehrere aus der Gruppe von Petrolether, Toluol, Xylol, Cyclohexan, Chlortoluol, Ligroin, 1,2-Dichlorethan, Triethylamin, Aceton, Acetonitril, Dimethylacetamid, Dimethylformamid, N-Methyl-pyrrolidon, N-Methyl-caprolactam, Tetraethylharnstoff, Dimethylsulfoxid, Diethylsulfon, Methyl-tert.-butylether, Anisol, Tetrahydrofuran, Methanol, Isopropanol und Wasser eingesetzt wird.

## Claims

1. Process for the preparation of 6-amino-nicotinonitriles of the formula 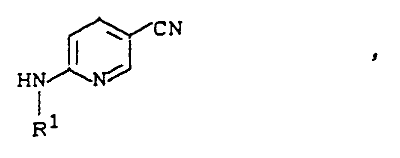 in which
R¹ denotes hydrogen, straight-chain or branched C₁-C₈-alkyl, C₃-C₈-alkenyl, C₂-C₈-alkoxyalkyl, C₄-C₈-alkoxyalkenyl, C₃-C₈-cycloalkyl, C₇-C₁₀-aralkyl or -N(R²R³),
where R² and R³, independently of one another, denote hydrogen, straight-chain or branched C₁-C₈-alkyl, phenyl, tolyl, C₇-C₁₀-aralkyl or a 5- to 8-membered saturated or unsaturated heterocyclic ring having 1 or 2 hetero atoms from the group consisting of N, O and S, where furthermore R² and R³ together with the N atom to which they are bonded can form a 5- to 8-membered ring which can contain a further hetero atom from the group consisting of N, O and S,
characterized in that methylene-glutacononitriles of the formula 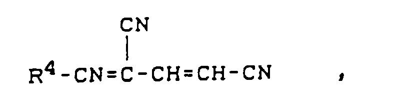 in which
R⁴ represents -OR⁵ or -N(R⁵, R⁶)
where R⁵ and R⁶ represent a straight-chain or branched C₁-C₈-alkyl, C₃-C₈-alkenyl, C₂-C₈-alkoxyalkenyl, C₄-C₈-alkoxyalkenyl, C₃-C₈-cycloalkyl or C₇-C₁₀-aralkyl, where furthermore R⁵ and R⁶ together with the N atom to which they are bonded can form a 5- to 8-membered ring which can contain a further hetero atom from the group consisting of N, O and S,
are reacted with nitrogen compounds of the formula
R¹-NH₂
in which
R¹ has the abovementioned meaning,
at a temperature from 0 to 200°C, preferably 20 to 150°C, and a molar ratio of nitrogen compound to glutacononitrile of 1 to 100:1, preferably 1 to 10:1, and in the presence or absence of an inert solvent.

2. Process according to Claim 1, characterized in that R⁴ is replaced by the substituent R¹⁴, which represents -N(R⁵, R⁶).

3. Process according to Claim 1, characterized in that R¹ is replaced by the substituent R¹¹, which represents hydrogen, straight-chain or branched C₁-C₈-alkyl, cyclopropyl, cyclopentyl, cyclohexyl or benzyl.

4. Process according to Claim 3, characterized in that R¹¹ is replaced by the substituent R²¹, which represents hydrogen, straight-chain or branched C₁-C₄-alkyl or benzyl.

5. Process according to Claim 1, characterized in that R² and R³ are replaced by the substituents R¹² and R¹³, respectively, which, independently of one another, represent hydrogen, straight-chain or branched C₁-C₈-alkyl, cyclopropyl, cyclopentyl, cyclohexyl, phenyl or benzyl, where both substituents R¹² and R¹³ together with the N atom to which they are bonded can form a 5- to 8-membered ring which can contain a further hetero atom from the group consisting of N, O and S.

6. Process according to Claim 5, characterized in that R¹² and R¹³ are replaced by the substituents R²² and R²³, which, independently of one another, represent hydrogen, C₁-C₄-alkyl, phenyl or benzyl and where furthermore the substituents R²² and R²³ together with the N atom to which they are bonded can form morpholine, pyrrolidine or piperidine, each of which can be substituted by C₁-C₄-alkyl or by hydroxy-C₁-C₄-alkyl.

7. Process according to Claim 1, characterized in that R⁵ and R⁶ are replaced by the substituents R¹⁵ and R¹⁶, which, independently of one another, represent straight-chain or branched C₁-C₈-alkyl, cyclopropyl, cyclopentyl, cyclohexyl, phenyl, benzyl or a 5- to 8-membered saturated or unsaturated heterocyclic ring which can contain a further hetero atom from the group consisting of N, O and S.

8. Process according to Claim 7, characterized in that R¹⁵ and R¹⁶ are replaced by the substituents R²⁵ and R²⁶, respectively, which, independently of one another, represent straight-chain or branched C₁-C₄-alkyl, phenyl or benzyl.

9. Process according to Claim 1, characterized in that all or some of the nitrogen compound is employed in the form of a salt of an acid.

10. Process according to Claim 1, characterized in that the solvent employed is one or more substances from the group consisting of hydrocarbons, halogenated hydrocarbons, tertiary amines, ketones, nitriles, dialkylcarboxamides, N-alkyl-lactams, peralkylureas, dialkyl sulphoxides, dialkyl sulphones, sulpholanes, ethers, peralkylphosphoramidates, alcohols, water, preferably one or more substances from the group consisting of petroleum ether, toluene, xylene, cyclohexane, chlorotoluene, ligroin, 1,2-dichloroethane, triethylamine, acetone, acetonitrile, dimethylacetamide, dimethylformamide, N-methylpyrrolidone, N-methyl-caprolactam, tetraethylurea, dimethyl sulphoxide, diethyl sulphone, methyl tert.-butyl ether, anisole, tetrahydrofuran, methanol, isopropanol and water.

## Revendications

1. Procédé pour la préparation de nitriles 6-aminonicotiniques de formule : 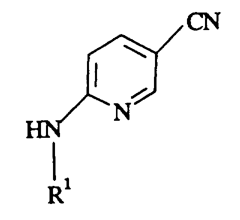 dans laquelle
R¹ représente l'hydrogène, un groupe alkyle en C₁-C₈ à chaîne droite ou ramifiée, alcényle en C₃-C₈, alcoxyalkyle en C₂-C₈, alcoxyalcényle en C₄-C₈, cycloalkyle en C₃-C₈, aralkyle en C₇-C₁₀ ou un groupe -N(R²R³) dans lequel
R² et R³ représentent chacun, indépendamment l'un de l'autre, l'hydrogène, un groupe alkyle à chaîne droite ou ramifiée en C₁-C₈, phényle, tolyle, aralkyle en C₇-C₁₀ ou un hétérocycle saturé ou insaturé de 5 à 8 chaînons contenant 1 ou 2 hétéroatomes choisis parmi N, O et S, R² et R³ pouvant également former ensemble et avec l'atome d'azote auquel ils sont reliés un cycle de 5 à 8 chaînons qui peut contenir un autre hétéroatome choisi parmi N, O et S,
ce procédé se caractérisant en ce que l'on fait réagir des dinitriles méthylèneglutaconiques de formule 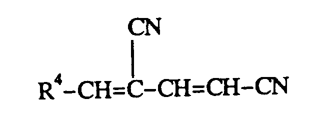 dans laquelle
R⁴ représente -OR⁵ ou -N(R⁵,R⁶),
dans lesquels R⁵ et R⁶ représentent chacun un groupe alkyle à - chaîne droite ou ramifiée en C₁-C₈, alcényle en C₃-C₈, alcoxyalcényle en C₂-C₈, alcoxyalcényle en C₄-C₈, cycloalkyle en C₃-C₈ ou aralkyle en C₇-C₁₀, R⁵ et R⁶ pouvant également former ensemble et avec l'atome d'azote auquel ils sont reliés un cycle de 5 à 8 chaînons qui peut contenir un autre hétéroatome choisi parmi N, O et S,
avec des composés azotés de formule
R¹-NH₂
dans laquelle
R¹ a les significations indiquées ci-dessus,
à des températures de 0 à 200°C, de préférence de 20 à 150°C, à un rapport molaire de 1 à 100:1, de préférence de 1 à 10:1 entre le composé azoté et le dinitrile glutaconique, et en présence ou en l'absence d'un solvant inerte.

2. Procédé selon la revendication 1, caractérisé en ce que, à la place de R⁴, il y a le substituant R¹⁴ consistant en un groupe -N(R⁵, R⁶).

3. Procédé selon la revendication 1, caractérisé en ce que, à la place de R¹, il y a le substituant R¹¹ qui consiste en l'hydrogène, un groupe alkyle à chaîne droite ou ramifiée en C₁-C₈, cyclopropyle, cyclopentyle, cyclohexyle ou benzyle.

4. Procédé selon la revendication 3, caractérisé en ce que, à la place de R¹¹, il y a le substituant R²¹ qui consiste en l'hydrogène, un groupe alkyle à chaîne droite ou ramifiée en C₁-C₄ ou un groupe benzyle.

5. Procédé selon la revendication 1, caractérisé en ce que, à la place de R² et R³, il y a les substituants R¹² et R¹³ qui consistent chacun, indépendamment l'un de l'autre, en l'hydrogène, un groupe alkyle à chaîne droite ou ramifiée en C₁-C₈, cyclopropyle, cyclopentyle, cyclohexyle, phényle ou benzyle, les deux substituants R¹² et R¹³ pouvant également former ensemble et avec l'atome d'azote auquel ils sont reliés un cycle de 5 à 8 chaînons qui peut contenir un autre hétéroatome choisi parmi N, O et S.

6. Procédé selon la revendication 5, caractérisé en ce que, à la place de R¹² et R¹³, il y a les substituants R²² et R²³ qui consistent chacun, indépendamment l'un de l'autre, en l'hydrogène, un groupe alkyle en C₁-C₄, phényle, benzyle, les substituants R²² et R²³ pouvant également former ensemble et avec l'atome d'azote auquel ils sont reliés un cycle morpholine, pyrrolidine ou pipéridine qui peut être substitué par un groupe alkyle en C₁-C₄ ou hydroxyalkyle en C₁-C₄.

7. Procédé selon la revendication 1, caractérisé en ce que, à la place de R⁵ et R⁶, il y a les substituants R¹⁵ et R¹⁶ qui consistent chacun, indépendamment l'un de l'autre, en un groupe alkyle à chaîne droite ou ramifiée en C₁-C₈, cyclopropyle, cyclopentyle, cyclohexyle, phényle, benzyle ou en un hétérocycle saturé ou insaturé de 5 à 8 chaînons qui peut contenir un autre hétéroatome choisi parmi N, O et S.

8. Procédé selon la revendication 7, caractérisé en ce que, à la place de R¹⁵ et R¹⁶, il y a les substituants R²⁵ et R²⁶ qui consistent chacun, indépendamment l'un de l'autre, en un groupe alkyle à chaîne droite ou ramifiée en C₁-C₄, phényle ou benzyle.

9. Procédé selon la revendication 1, caractérisé en ce que le composé azoté est mis en oeuvre en totalité ou en partie à l'état de sel d'acide.

10. Procédé selon la revendication 1, caractérisé en ce que l'on utilise un ou plusieurs solvants choisis parmi les hydrocarbures, les hydrocarbures halogénés, les amines tertiaires, les cétones, les nitriles, les dialkylcarboxamides, les N-alkyllactames, les peralkylurées, les dialkylsulfoxydes, les dialkylsulfones, les sulfolanes, les éthers, les peralkylamides phosphoriques, les alcools, l'eau, de préférence un ou plusieurs solvants choisis parmi l'éther de pétrole, le toluène, le xylène, le cyclohexane, le chlorotoluène, l'essence minérale légère, le 1,2-dichloréthane, la triéthylamine, l'acétone, l'acétonitrile, le diméthylacétamide, le diméthylformamide, la N-méthylpyrrolidone, le N-méthylcaprolactame, la tétraéthylurée, le diméthylsulfoxyde, la diéthylsulfone, l'oxyde de méthyle et de tert-butyle, l'anisole, le tétrahydrofurane, le méthanol, l'isopropanol et l'eau.
